(19) Europäisches Patentamt
European Patent Office
Office européen des brevets

(11) Publication number: **0 150 609**
**A2**

(12) # EUROPEAN PATENT APPLICATION

(21) Application number: 84308941.8

(22) Date of filing: 20.12.84

(51) Int. Cl.⁴: C 07 D 501/20, A 61 K 31/545

(30) Priority: 30.12.83 GB 8334598
30.12.83 GB 8334601

(43) Date of publication of application: 07.08.85
Bulletin 85/32

(84) Designated Contracting States: AT BE CH DE FR GB IT LI LU NL SE

(71) Applicant: GLAXO GROUP LIMITED, Clarges House 6-12 Clarges Street, London W1Y 8DH (GB)

(72) Inventor: Foxton, Michael Walter, The Finches Mill Lane, Chalfont St.Giles Buckinghamshire (GB)
Inventor: Looker, Brian Edgar, 28 Middleton Avenue, Greenford Middlesex (GB)

(74) Representative: Holmes, Michael John et al, Frank B. Dehn & Co. European Patent Attorneys Imperial House 15-19 Kingsway, London, WC2B 6UZ, (GB)

(54) **Cephalosporin antibiotics.**

(57) Cephalosporin compounds of general formula (I)

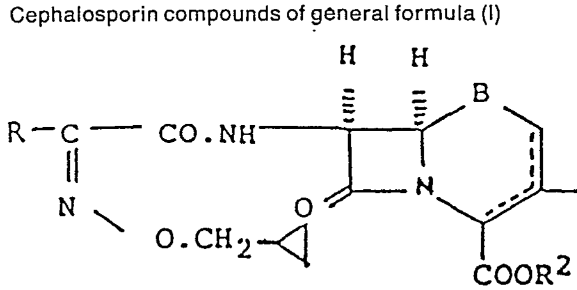

(I)

(wherein R represents optionally protected aminopyrimidyl or 5-amino-1,2,4-thiadiazol-3-yl,

$R^2$ represents hydrogen or a carboxyl blocking group,

Y represents hydrogen, halogen, $C_{1-4}$ alkoxy or alkylthio, methyl, ethyl, vinyl, acetoxymethyl, optionally N-substituted carbamoyloxymethyl, heterocyclicthiomethyl or optionally substituted pyridiniummethyl or bicyclicpyridiniummethyl, B represents –S– or >S → O, and the dotted line indicates $\Delta^2$ or $\Delta^3$ unsaturation) and salts thereof.

The $\Delta^3$ sulphide compounds exhibit a particularly advantageous profile of antibiotic activity against both Gram-positive and Gram-negative organisms, including β-lactamase-producing strains.

Methods of preparing the compounds of the invention are described, as is the use of the compounds as active antibiotics and as precursors therefor.

EP 0 150 609 A2

0150609

FE 146-973

# Cephalosporin Antibiotics

This invention relates to improvements in or relating to cephalosporins. More particularly it relates to new cephalosporin compounds and derivatives thereof having valuable antibiotic activity.

The cephalosporin compounds in this specification are named with reference to "cepham" after J.Amer.Chem. Soc., 1962, 84, 3400, the term "cephem" referring to the basic cepham structure with one double bond.

Cephalosporin antibiotics are widely used in the treatment of diseases caused by pathogenic bacteria in human beings and animals, and are especially useful in the treatment of diseases caused by bacteria which are resistant to other antibiotics such as penicillin compounds, and in the treatment of penicillin-sensitive patients. In many instances it is desirable to employ a cephalosporin antibiotic which exhibits activity against both Gram-positive and Gram-negative microorganisms, and a significant amount of research has been directed to the development of various types of broad spectrum cephalosporin antibiotics.

Thus, for example in our British Patent Specification No. 1399086, we describe a novel class of cephalosporin antibiotics containing a 7β-(α-etherified oxyimino)acylamido group, the oxyimino group having the syn configuration. This class of antibiotic compounds is characterised by high antibacterial activity against a range of Gram-positive and Gram-negative organisms coupled with particularly high stability to β-lactamases produced by various Gram-negative organisms.

The discovery of this class of compounds has stimulated further research in the same area in attempts to find compounds which have improved properties, for example against particular classes of organisms especially Gram-negative organisms.

This interest is reflected in the very large numbers of patent applications which have been filed relating to cephalosporin antibiotics having particular oxyimino etherifying groups in combination with particular substituents both on the 7β-acylamido side chain and at the 3-position of the cephalosporin nucleus.

In European Patent Application No. 0013762 a wide variety of cephalosporin compounds are generically defined in which the 7β-side chain may be selected from <u>inter alia</u>, a 2-(5-amino-1,2,4-thiadiazol-3-yl)-2-(etherified oxyimino)acetamido group, in which the etherifying group, amongst many possible meanings, may be a substituted lower alkyl group. However, the list of suitable substituents does not include cycloalkyl, nor is there any indication that such a substituent may be employed. The 3-position group may also be selected from a large number of alternatives.

In European Patent Application No. 0046964 and British Patent Application No. 2010840 A a wide variety of cephalosporin compounds are generically defined in which the 7β-side chain may be selected from <u>inter alia</u>, a 2-(aminopyrimidyl)-2-(etherified oxyimino)acetamido group, in which the etherifying group, amongst many possible meanings, may be an alkyl group which may optionally be substituted. According to these specifications, the 3-position group may also be selected from a large number of alternatives. It should be noted that there is no indication that the etherified oxyimino group can be an alkoxyimino group carrying a cyclopropyl group or, indeed, any other cycloalkyl group.

We have now discovered that by the selection of a (Z)-2-(etherified oxyimino)acetamido group at the 7β-position (the said group being substituted at the 2-position thereof by certain particular heterocyclic groups as herein defined) in combination with certain particular groups as herein defined

at the 3-position of the cephalosporin nucleus, and by the selection of a cyclopropylmethoxyimino group as the etherified oxyimino grouping, cephalosporin compounds having a particularly advantageoous profile of activity (described in more detail below) against a wide range of commonly encountered pathogenic organisms may be obtained.

According to one aspect of the invention we provide cephalosporin compounds of general formula (I)

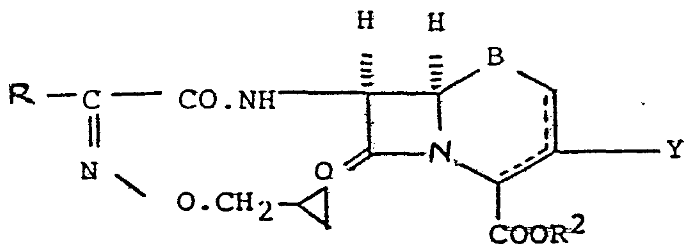

(I)

and salts thereof,
wherein R represents a group of formula

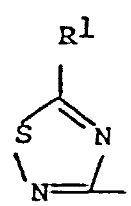

or

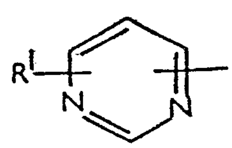

(wherein $R^1$ represents an amino or protected amino group);
$R^2$ represents a hydrogen atom or a carboxyl blocking group;
B is -S- or >S→O (α- or β-);
the dotted line bridging the 2-, 3- and 4-positions indicates that the compound is a ceph-2-em or ceph-3-em compound; and
Y represents a hydrogen or halogen atom; a $C_{1-4}$ alkoxy group; a methyl, ethyl or vinyl group; a

$C_{1-4}$ alkylthio group; an acetoxymethyl group; a carbamoyloxymethyl group which may optionally be substituted on the nitrogen atom by a $C_{1-4}$ alkyl or $C_{1-4}$ haloalkyl group; an N-protected carbamoyloxymethyl group; a group of formula $-CH_2SZ$ (wherein Z is a carbon-linked 5- or 6-membered unsaturated heterocyclic ring containing at least one nitrogen atom, which ring may also contain one or more sulphur atoms and/or may be substituted by one or more substituents independently selected from $C_{1-4}$ alkyl, oxo, hydroxy and carbamoylmethyl groups); a pyridiniummethyl group which may optionally be substituted by one or more substituents independently selected from halogen atoms and carbamoyl, hydroxymethyl and $C_{1-4}$ alkoxy groups; or a bicyclic pyridiniummethyl group of formula

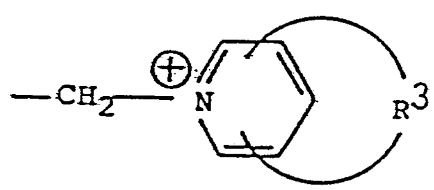

(wherein $R^3$ represents an optionally substituted $C_{2-5}$ hydrocarbon chain which is saturated or contains one or two double bonds and which may optionally be interrupted by a heteroatom), with the proviso that, when R represents a 5-amino-1,2,4-thiadiazol-3-yl group, the group $-COOR^2$ is a carboxyl group, B is -S- and the dotted line represents $\Delta^3$ unsaturation, Y does not represent a 2,3-cyclopentenopyridiniummethyl group.

It will be understood that the term "salt" used herein, both in connection with compounds of general formula I and with intermediates used in their preparation, includes within its scope external and internal salts. External salts may, for example, be base salts or may be acid addition salts formed with e.g. a mineral or organic acid.

Internal salts may for example take the form of zwitterions or betaines. For pharmaceutical use, salts will be non-toxic salts but other salts may find use, for example in the preparation of compounds to be used as pharmaceuticals.

It will be appreciated that the group Y may carry a positive charge, e.g. when Y is a pyridiniummethyl or bicyclic pyridiniummethyl group, and when this is the case the positive charge must be balanced by a negative charge. Thus the compounds may be betaines, so that the negative charge is provided by a $-COO^{\ominus}$ group at the 4-position. Alternatively, the negative charge may be provided by an anion $A^{\ominus}$. In compounds to be used in medicine such an anion will be non-toxic and may be derived from any of the acids described below which will form non-toxic salt derivatives.

The compounds according to the invention are *syn* isomers. The *syn* isomeric form is defined by the configuration of the

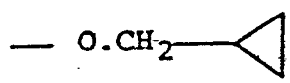

group with respect to the carboxamido group. In this Specification, the *syn* configuration is denoted structurally as

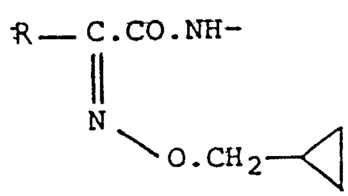

It will be understood that since the compounds according to the invention are geometric isomers, some admixture with the corresponding anti isomer may occur.

The invention also includes within its scope the solvates (especially the hydrates) of the compounds of formula (I). It also includes within its scope the solvates of salts of compounds of formula (I). It will be appreciated that where the compounds are to be used in medicine, the solvates should be pharmacologically acceptable.

When the group Y in the compounds of the invention represents a $C_{1-4}$ alkoxy or $C_{1-4}$ alkylthio group, this may for example be an ethoxy or a methylthio group. When the group Y represents a group of formula $-CH_2SZ$, the heterocyclic ring represented by Z may for example contain 1 to 4 nitrogen atoms and, if desired, a sulphur atom; particular examples of these heterocyclic groups include imidazolyl, pyrazolyl, pyridyl, pyrimidyl, pyrazinyl, pyridazinyl, triazolyl, tetrazolyl, thiazolyl, thiadiazolyl, triazinyl and thiazolidinyl. The heterocylcic ring may, if desired, be substituted by one or more (for example, one to three) $C_{1-4}$ alkyl (e.g. methyl), oxo, hydroxy or carbamoylmethyl groups. Alkyl or carbamoylmethyl substituents may be attached for example, to the nitrogen heteroatom(s). In the heterocyclic ring represented by Z one nitrogen atom may be quaternised. Examples of the group Z include a 1-methylpyridinium-2-yl, 1-methylpyridinium-3-yl, 1-methylpyridinium-4-yl, 1-methyltetrazol-5-yl, 1,2-dimethylpyrazolium-3-yl, 1,3-dimethylimidazolium-3-yl, 1-methylpyrimidinium-2-yl, 1-carbamoylmethylpyridinium-4-yl, 2,5-dihydro-6-hydroxy-2-methyl-5-oxo-1,2,4-triazin-3-yl, or 4,5-dihydro-6-hydroxy-4-methyl-5-oxo-1,2,4-triazin-3-yl group.

When Y represents a bicyclic pyridinium group in which the $C_{2-5}$ hydrocarbon chain is interrupted by a heteroatom, the said heteroatom may, for example, be an oxygen, nitrogen or sulphur atom. When the group Y represents a bicyclic pyridiniummethyl group, this may for example by a 2,3-cyclopentenopyridiniummethyl group.

When the group Y in the compounds of the invention represents an N-protected carbamoyloxymethyl group, this group Y may, for example, be a group of formula $-CH_2.O.CO.NHR^3$ where $R^3$ is a conventional N-protecting group such as, for example, a labile group such as an acyl group, especially a lower alkanoyl group such as acetyl, a halo-substituted lower alkanoyl group such as mono-, di- or trichloroacetyl, a chloro- or bromo-sulphonyl group, or a halogenated alkoxycarbonyl group such as 2,2,2-trichloroethoxycarbonyl.

When the group $R^1$ in the compounds of the invention represents a protected amino group, this may, for example, be an amino group protected using a conventional protecting group, as discussed in more detail below.

When the group $R^2$ in the compounds of the invention represents a carboxyl blocking group, this may for example be the residue of an ester-forming aliphatic or araliphatic alcohol or an ester-forming phenol, silanol or stannanol (the said alcohol, phenol, silanol or stannanol preferably containing 1 to 20 carbon atoms) or a symmetrical or mixed anhydride blocking group derived from an appropriate acid. Where the compound is to be used in medicine any ester function $R^2$ should be a non-toxic metabolically labile ester function, as discussed in more detail below.

Ceph-3-em compounds of formula (I) in which $R^1$ is an amino group, $R^2$ is a hydrogen atom or non-toxic metabolically labile ester function,

and B is -S-, as well as the non-toxic salts thereof, exhibit interesting biological properties (as described in detail below) and are therefore preferred compounds of the invention. These compounds may be represented by the general formulae

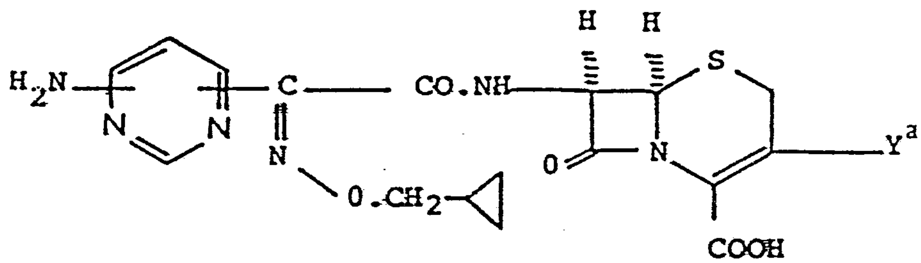

(Ia)

and

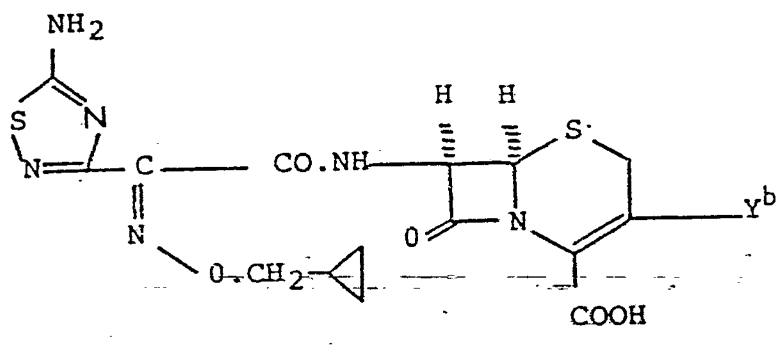

(Ib)

(wherein $Y^a$ is as defined above for Y other than an N-protected carbamoyloxymethyl group and $Y^b$ is as defined above for Y other than a 2,3-cyclopentenopyridiniummethyl or an N-protected carbamoyloxymethyl group) and non-toxic salts (including zwitterionic and betaine forms) and non-toxic metabolically labile esters thereof. Other compounds according to the invention, e.g. compounds in which $R^1$ is a protected amino group, may however be intermediates useful in the preparation of the active compounds.

The preferred compounds of general formula Ia as defined above include those in which the aminopyrimidyl group of the 7β-side chain is a 4-aminopyrimid-2-yl group.

Of the compounds of general formula Ib as defined above, there may be particularly mentioned those wherein $Y^b$ represents a hydrogen or halogen atom; a $C_{1-4}$ alkoxy group; a methyl, ethyl or vinyl group; a $C_{1-4}$ alkylthio group; an acetoxymethyl group; a carbamoyloxy group which may optionally be substituted on the nitrogen atom by a $C_{1-4}$ alkyl or $C_{1-4}$ haloalkyl group; a group of formula $-CH_2SZ$ (wherein Z is as defined above); or a pyridiniummethyl group which may optionally be substituted by one or more substituents independently selected from carbamoyl, hydroxymethyl and $C_{1-4}$ alkoxy groups.

Particularly preferred compounds of general formulae Ia and Ib as defined above are those in which $Y^a$ and $Y^b$ each represents a pyridiniummethyl or a carbamoyloxymethyl group.

Intermediates such as e.g. ceph-2-em, sulphoxide and protected intermediates are useful in the preparation of the active compounds of the invention.

The compounds according to the present invention may exist in tautomeric forms (for example in respect of the 5-aminothiadiazolyl group or the aminopyrimidyl group or in respect of a 3-substituent such as a 2,5-dihydro-6-hydroxy-2-methyl-5-oxo-1,2,4-triazin-3-ylthiomethyl group) and it will be understood that such tautomeric forms are included within the scope of the invention.

Examples of non-toxic metabolically labile ester derivatives include acyloxyalkyl esters, e.g. lower alkanoyloxy-methyl or -ethyl esters such as acetoxy-methyl or -ethyl or pivaloyloxymethyl esters, and alkoxycarbonyloxyalkyl esters, e.g. lower alkoxycarbonyloxyethyl esters such as the ethoxycarbonyloxyethyl ester. In addition to the

above ester derivatives, the present invention includes within its scope the active compounds of the invention in the form of other physiologically acceptable equivalents, i.e. physiologically acceptable compounds which, like the metabolically labile esters, are converted in vivo into the parent antibiotic compounds of the invention.

Non-toxic salt derivatives which may be formed by reaction of the carboxyl group present in the compounds of formula (I) or by reaction of any acidic hydroxyl group which may be present on the group Y, include inorganic base salts such as alkali metal salts (e.g. sodium and potassium salts) and alkaline earth metal salts (e.g. calcium salts); amino acid salts (e.g. lysine and arginine salts) and other organic base salts (e.g. procaine, phenylethylbenzylamine, dibenzylethylenediamine, ethanolamine, diethanolamine and N-methylglucosamine salts). Other non-toxic salt derivatives include acid addition salts, e.g. formed with hydrochloric, hydrobromic, sulphuric, nitric, phosphoric, formic and trifluoroacetic acids. Where appropriate, the compounds may also, as indicated above, be in zwitterionic, i.e. internal salt, form. The salts may also be in the form of resinates formed with, for example, a polystyrene resin or cross-linked polystyrene divinylbenzene copolymer resin containing amino or quaternary amino groups or sulphonic acid groups, or with a resin containing carboxyl groups, e.g. a polyacrylic acid resin. Soluble base salts (e.g. alkali metal salts such as the sodium salt) of the compounds of formula (I) may be used in therapeutic applications because of the rapid distribution of such salts in the body upon administration. Where, however, insoluble salts of compounds (I) are desired in a particular application, e.g. for use in depot preparations, such salts may be formed in conventional manner, for example with appropriate organic amines.

Active compounds according to the invention exhibit broad spectrum antibiotic activity both *in vitro* and *in vivo*. They have good activity against both Gram-positive and Gram-negative organisms, including many β-lactamase producing strains. The compounds also possess high stability to β-lactamases produced by a range of Gram-negative and Gram-positive organisms.

Active compounds according to the invention have been found to exhibit good activity against strains of *Staphylococcus aureus* and *Staphylococcus epidermidis* species including penicillinase producing strains of these Gram-positive bacteria. This is coupled with good activity against various members of the Enterobacteriaceae (e.g. strains of *Escherichia coli*, *Klebsiella pneumoniae*, *Enterobacter cloacae*, *Serratia marcescens*, *Proteus mirabilis* and indole-positive *Proteus* organisms such as *Proteus vulgaris*, *Proteus morganii* and *Providence* species), and strains of *Haemophilus influenzae* and *Acinetobacter calco-aceticus* as well as activity against strains of *Pseudomonas* species. This combination of high activity against Gram-positive organisms with high activity against Gram-negative organisms possessed by compounds of the invention is unusual and particularly advantageous.

Active compounds of the invention may be used for treating a variety of diseases caused by pathogenic bacteria in human beings and animals, such as respiratory tract infections and urinary tract infections.

According to another embodiment of the invention we provide a process for the preparation of cephalosporin compounds of general formula (I) and salts thereof, which comprises

(A) acylating a compound of the formula (II)

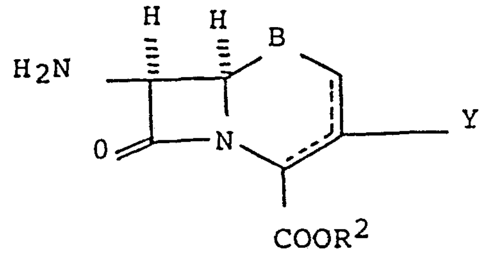

(II)·

(wherein Y, B, the dotted line and $R^2$ are as defined above) or a salt (including zwitterionic and betaine forms) thereof, e.g. an acid addition salt (formed with, for example, a mineral acid such as hydrochloric, hydrobromic, sulphuric, nitric or phosphoric acid or an organic acid such as methanesulphonic or toluene-4-sulphonic acid) or a 7-N-silyl derivative thereof, with an acid of formula (III)

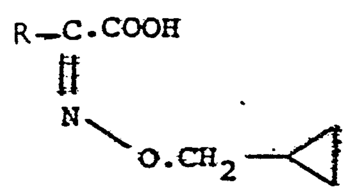

(III)

(wherein R is as defined above) or a salt thereof, or with an acylating agent corresponding thereto;

(B) where Y in the compound of the invention represents an acetoxymethyl or optionally substituted carbamoyloxymethyl group, reacting a compound of formula (IV)

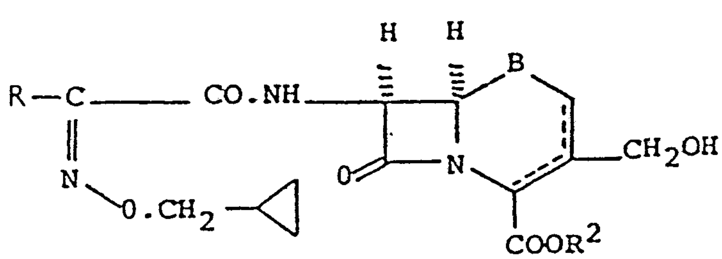

(IV)

(where R, $R^2$, B and the dotted line are as defined above) or a salt thereof, with an acylating agent serving to form the group $-CH_2Y'$ at the 3-position (wherein $Y'$ is an acetoxy group; a carbamoyloxy group optionally substituted on the nitrogen atom by a $C_{1-4}$ alkyl or $C_{1-4}$ haloalkyl group; or an N-protected carbamoyloxy group such as a group of formula $-O.CO.NHR^3$ where $R^3$ is an N-protecting group, e.g. a labile group as described above);

(C) where Y in the compound of the invention represents an optionally substituted pyridiniummethyl or bicyclic pyridiniummethyl group or a group of formula $-CH_2SZ$ (where Z is as defined above), reacting a compound of formula (V)

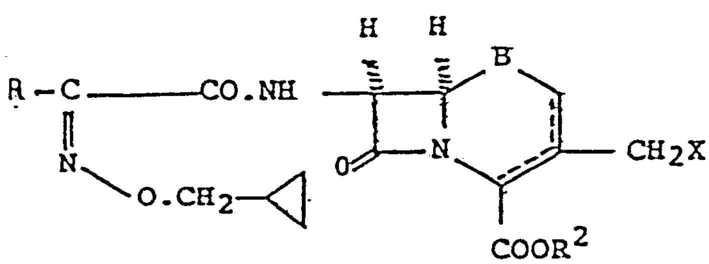

(V)

(wherein R, $R^2$, B and the dotted line are as hereinbefore defined, and X respresents a replaceable residue of a nucleophile e.g. an acetoxy or dichloro-acetoxy group or a halogen atom such as chlorine, bromine or iodine) or a salt thereof, with a nucleophile serving to form the desired group Y at the 3-position; or

(D) where Z in the compound of the invention contains a $C_{1-4}$ alkyl-substituted or carbamoylmethyl-substituted nitrogen atom in the heterocyclic ring, reacting a compound of formula (I')

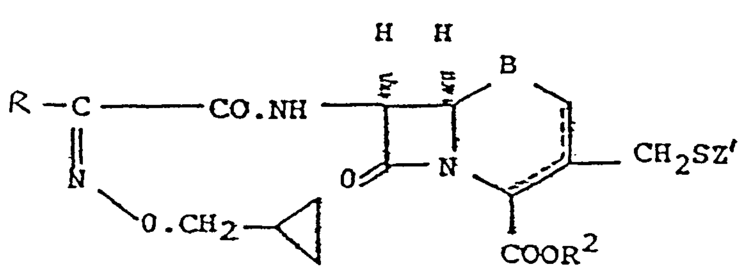

(wherein R, B and the dotted line are as defined above; $R^2$ in this instance represents a carboxyl blocking group; and Z' represents a carbon-linked 5- or 6-membered heterocyclic ring containing a nitrogen atom) with a $C_{1-4}$ alkylating agent or a carbamoylmethylating agent serving to introduce a $C_{1-4}$ alkyl group or a carbamoylmethyl group as a substituent on the nitrogen atom in the heterocyclic ring of Z'.

After any of the above processes (A) to (D) any of the following reactions, in any appropriate sequence, may be carried out to give compounds of formula (Ia) or (Ib) or non-toxic salts and non-toxic metabolically labile esters thereof:-

i) conversion of a $\Delta^2$-isomer into a $\Delta^3$-isomer,

ii) reduction of a compound wherein B is $>S \rightarrow O$ to form a compound wherein B is $-S-$,

iii) conversion of a carboxyl group into a non-toxic metabolically labile ester function,

iv) formation of a non-toxic salt, and

v) removal of any carboxyl blocking and/or N-protecting groups.

In the above-described process (A), the starting material of formula ~~(II) is preferably~~ a compound wherein B is -S- and the dotted line represents a ceph-3-em compound.

When the group Y in formula (II) is charged e.g. when Y represents a pyridiniummethyl group, and the compound contains a group of formula $-COOR^2$ (wherein $R^2$ is as defined above) at the 4-position, the compound will include an associated anion $E^{\ominus}$ such as a halide, e.g. chloride or bromide, or trifluoroacetate anion.

Acylating agents which may be employed in the preparation of compounds of formula (I) include acid halides, particularly acid chlorides or bromides. Such acylating agents may be prepared by reacting

an acid (III) or a salt thereof with a halogenating agent e.g. phosphorus pentachloride, thionyl chloride or oxalyl chloride.

Acylations employing acid halides may be effected in aqueous and non-aqueous reaction media, conveniently at temperatures of from -50 to +50°C, preferably -40 to +30°C, if desired in the presence of an acid binding agent. Suitable reaction media include aqueous ketones such as aqueous acetone, aqueous alcohols such as aqueous ethanol, esters such as ethyl acetate, halogenated hydrocarbons such as methylene chloride, amides such as dimethylacetamide, nitriles such as acetonitrile, or mixtures of two or more such solvents. Suitable acid binding agents include tertiary amines (e.g. triethylamine or dimethylaniline), inorganic bases (e.g. calcium carbonate or sodium bicarbonate), and oxiranes such as lower 1,2-alkylene oxides (e.g. ethylene oxide or propylene oxide) which bind hydrogen halide liberated in the acylation reaction.

Acids of formula (III) may themselves be used as acylating agents in the preparation of compounds of formula (I). Acylations employing acids (III) are desirably conducted in the presence of a condensing agent, for example a carbodiimide such as N,N'-dicyclohexylcarbodiimide or N-ethyl-N'-γ-dimethylaminopropylcarbodiimide; a carbonyl compound such as carbonyldiimidazole; or an isoxazolium salt such as N-ethyl-5-phenylisoxazolium perchlorate.

Acylation may also be effected with other amide-forming derivatives of acids of formula (III) such as, for example, an activated ester, a symmetrical anhydride or a mixed anhydride (e.g. formed with pivalic acid or with a haloformate, such as a lower alkylhaloformate). Mixed anhydrides may also be formed with phosphorus acids (for example phosphoric or phosphorous acids), sulphuric acid or aliphatic or aromatic sulphonic acids (for example toluene-4-sulphonic acid). An activated ester may conveniently be formed *in situ* using, for example, 1-hydroxybenzotriazole in the presence of a condensing agent as set out above. Alternatively, the activated ester may be preformed.

Acylation reactions involving the free acids or their above-mentioned amide-forming derivatives are desirably effected in an anhydrous reaction medium, e.g. methylene chloride, tetrahydrofuran, dimethylformamide or acetonitrile.

An alternative method of activation is, for example, by reacting an acid of formula (III) with a solution or suspension preformed by adding a carbonyl halide, in particular oxalyl chloride or phosgene, or a phosphoryl halide such as phosphorous oxychloride to a solvent such as a halogenated hydrocarbon, for example methylene chloride, containing a lower acyl tertiary amide such as N,N-dimethylformamide. The activated form of the the acid of formula (III) may then be reacted with a 7-amino compound of formula (II) in a suitable solvent or mixture of solvents for example a halogenated hydrocarbon e.g. dichloromethane. The acylation reaction may conveniently be effected at temperatures of from -50° to +50°C, preferably -40° to +30°C, if desired in the presence of an acid binding agent, for example as described above (e.g. dimethylaniline).

If desired, the above acylation reactions may be carried out in the presence of a catalyst such as 4-dimethylaminopyridine.

The acids of formula (III) and acylating agents corresponding thereto may, if desired, be prepared and employed in the form of their acid addition salts. Thus, for example, acid chlorides may conveniently be employed as their hydrochloride salts, and acid bromides as their hydrobromide salts.

Acetylation of 3-hydroxymethyl compounds of formula (IV) according to process (B) may be effected by conventional methods, for example in an analogous manner to that described in British Patent Specification No. 1141293, i.e. by blocking the 4-carboxy group, acetylating the 3-hydroxymethyl group of the protected compound and subsequently removing the blocking group.

Carbamoylation of 3-hydroxymethyl compounds of formula (IV) may be effected by conventional methods using suitable acylating (i.e. carbamoylating) agents. Suitable carbamoylating agents include isocyanates of formula $R^4.NCO$ (wherein $R^4$ is a labile substituent group or the group $R^3$ as defined above), to give a compound containing a 3-position substituent having the formula $-CH_2O.CONHR^4$ (wherein $R^4$ has the above defined meaning). The carbamoylation reaction may desirably be effected in the presence of a solvent or solvent mixture selected from hydrocarbons (e.g. aromatic hydrocarbons such as benzene and toluene), halogenated hydrocarbons (e.g. dichloromethane), amides (e.g. formamide or dimethylformamide), esters (e.g. ethyl acetate), ethers (e.g. cyclic ethers such as tetrahydrofuran and dioxan), ketones (e.g. acetone), sulphoxides (e.g. dimethylsulphoxide) and mixtures of these solvents. The reaction may conveniently be carried out at a temperature of between -80°C and the boiling temperature of the reaction mixture, for example up to 100°C, preferably between -20° and +30 °C. The labile group $R^4$ may subsequently be cleaved, e.g. by hydrolysis, to form a 3-carbamoyloxymethyl group. Examples of labile groups $R^4$ which are readily cleavable upon subsequent treatment include those labile groups hereinbefore given as examples of the group $R^3$. Such labile groups may generally be cleaved by acid or base catalysed hydrolysis (e.g. by base catalysed hydrolysis using sodium bicarbonate).

Halogenated groups such as chlorosulphonyl, dichlorophosphoryl, trichloroacetyl and 2,2,2-trichloroethoxycarbonyl may also be cleaved reductively, while groups such as chloroacetyl may also be cleaved by treatment with thioamides such as thiourea.

The carbamoylating agent is desirably used in excess (for example at least 1.1 moles relative to the compound of formula (IV)). The carbamoylation may be assisted by the presence of base, e.g. a tertiary organic base such as a tri-(lower alkyl)amine (e.g. triethylamine) or by employing the compound (IV) in the form of an alkali metal (e.g. sodium) salt, although such assistance may not be necessary in the case of more active isocyanates, e.g. compounds when $R^4$ is a strongly electron-withdrawing group such as chlorosulphonyl or trichloroacetyl. Carbamoylations involving reaction of a free acid of formula (IV) with excess isocyanate wherein $R^4$ is a group such as chlorosulphonyl or trichloroacetyl are thus of particular practical advantage by virtue of the simplicity of the reaction conditions, since there is no need for temporary blocking and subsequent deblocking of the 4-position carboxyl group of the cephalosporin and since the electron-withdrawing $R^4$ group in the resulting N-protected 3-carbamoyloxymethyl cephalosporin product is readily removed by, for example, hydrolysis with aqueous sodium bicarbonate.

It should be noted that it may be convenient to retain or even introduce an N-substituting group $R^4$ during transformations of intermediate 3-carbamoyloxymethyl compounds in order to minimise unwanted side reactions involving the carbamoyloxymethyl group.

Another useful carbamoylating agent is cyanic acid, which is conveniently generated *in situ*, for example, from an alkali metal cyanate such as sodium cyanate, the reaction being facilitated

by the presence of an acid, e.g. a strong organic acid such as trifluoroacetic acid. Cyanic acid effectively corresponds to the isocyanate compounds mentioned above wherein $R^4$ is hydrogen and therefore converts compounds of formula (IV) directly to their 3-carbamoyloxymethyl analogues.

Alternatively, carbamoylation may be effected by reaction of the compound of formula (IV) with phosgene or carbonyldiimidazole followed by ammonia or the appropriate substituted amine, optionally in an aqueous or non-aqueous reaction medium.

3-Hydroxymethyl starting materials of formula (IV) are novel compounds and form a further feature of the invention. They may be prepared by methods analogous to those described in British Patent No. 1474519 and U.S. Patent No. 3976546. Alternatively they may be prepared by N-acylating the corresponding 7-amino-3-hydroxymethyl compounds, e.g. analogously to process (A) above.

In process (C) above, the nucleophile may be used to displace a wide variety of substituents X from the cephalosporin of formula (V). To some extent the facility of the displacement is related to the pKa of the acid HX from which the substituent is derived. Thus, atoms or groups X derived from strong acids tend, in general, to be more easily displaced than atoms or groups derived from weaker acids. The facility of the displacement is also related, to some extent, to the precise character of the nucleophile. A sulphur nucleophile for introducing the group -SZ may be employed for example in the form of an appropriate thione, thiol or thiolate salt.

The displacement of X by the nucleophile may conveniently be effected by maintaining the reactants in solution or suspension. The reaction is advantageously effected using 1-10 molar equivalents of the nucleophile.

Nucleophilic displacement reactions may conveniently be carried out on those compounds of formula (V) wherein the substituent X is a halogen atom or an acyloxy group, for example as discussed below.

Acyloxy groups

Compounds of formula (V) wherein X is an acetoxy group are convenient starting materials for use in the nucleophilic displacement reaction with the nucleophile. Alternative starting materials in this class include compounds of formula (V) in which X is the residue of a substituted acetic acid e.g. chloroacetic acid, dichloroacetic acid and trifluoroacetic acid.

Displacement reactions on compounds (V) possessing X substituents in this class, particularly in the case where X is an acetoxy group, may be facilitated by the presence in the reaction medium of iodide or thiocyanate ions. For example, reactions of this type employing pyridine (including bicyclic pyridine) nucleophiles are described in more detail in British Patent Specifications Nos. 1132621 and 1171603 and British Patent Application 2098216A.

The substituent X may also be derived from formic acid, a haloformic acid such as chloroformic acid, or a carbamic acid.

When using a compound of formula (V) in which X represents an acetoxy or substituted acetoxy group, it is generally desirable that the group $R^2$ in formula (V) should be a hydrogen atom and that B should represent -S-. In this case, the reaction is advantageously effected in an aqueous medium. When using a pyridine nucleophile, the aqueous medium is preferably at a pH of 5 to 8, particularly 5.5 to 7.

Under aqueous conditions, the pH value of the reaction solution when employing a sulphur nucleophile is advantageously maintained in the range 6-8, if necessary by the addition of a base.

The base is conveniently an alkali metal or alkaline earth metal hydroxide or bicarbonate such as sodium hydroxide or sodium bicarbonate.

A process employing a pyridine nucleophile and a compound of formula (V) in which X is the residue of a substituted acetic acid may be carried out as described in British Patent Specification No. 1241657.

When using compounds of formula (V) in which X is an acetoxy group, the reaction is conveniently effected at a temperature of 10° to 110°C, preferably 20° to 80°C.

Halogens

Compounds of formula (V) in which X is a chlorine, bromine or iodine atom are novel compounds which form a further feature of the invention and can be conveniently used as starting materials in the nucleophilic displacement reaction with the nucleophile. When using compounds of formula (V) in this class, $R^2$ may conveniently represent a carboxyl blocking group. When using a sulphur nucleophile, B in the compound of formula (V) conveniently represents -S-, whereas when using a pyridine nucleophile, B is preferably $>S\rightarrow O$. The reaction is conveniently effected in a non-aqueous medium which preferably comprises one or more organic solvents, advantageously of a polar nature such as ethers, e.g. dioxan or tetrahydrofuran, esters, e.g. ethyl acetate, amides e.g. formamide and N,N-dimethylformamide, and ketones e.g. acetone. In some cases the nucleophile itself may be the solvent. Other suitable organic solvents are described in more detail in British Patent Specification No. 1326531. The reaction medium should be neither extremely acidic nor extremely basic.

In the case of reactions carried out on compounds of formula (V) in which $R^2$ is a carboxyl blocking group and the resulting Y group contains a quaternary

nitrogen atom, the product will be formed as the corresponding halide salt which may, if desired, be subjected to one or more ion exchange reactions to obtain a salt having the desired anion.

When using compounds of formula (V) in which X is a halogen atom as described above, the reaction is conveniently effected at a temperature of -20° to +60°, preferably 0° to +30°C.

When the incoming nucleophile does not yield a compound containing a quaternised nitrogen atom, the reaction is generally effected in the presence of an acid scavenging agent for example a base such as triethylamine or calcium carbonate.

In process (D) above, the compound of formula (I') is advantageously reacted with a compound of the formula $R^5W$ wherein $R^5$ is a $C_{1-4}$ alkyl group or a group of formula $H_2NCOCH_2$- and W is a leaving group such as a halogen atom (e.g. iodine, chlorine or bromine) or a hydrocarbylsulphonate (e.g. mesylate or tosylate) group. Alternatively, a di-$C_{1-4}$-alkyl sulphate, e.g. dimethyl sulphate, may be employed as an alkylating agent. Iodomethane is a preferred alkylating agent and iodoacetamide is a preferred carbamoylmethylating agent. The reaction is preferably carried out at a temperature in the range of 0 to 60°C, advantageously 20 to 30°C. Where the alkylating agent is liquid under the reaction conditions, as in the case of iodomethane, this agent can itself serve as a solvent. Alternatively, the reaction may be conveniently effected in an inert solvent such as an ether e.g. tetrahydrofuran, an amide, e.g. dimethylformamide, a lower alkanol, e.g. ethanol, a lower dialkylketone, e.g. acetone, a halogenated hydrocarbon e.g. dichloromethane or an ester e.g. ethyl acetate.

The compound of formula (I') used as starting material in process (D) is a compound according to the present invention and may be prepared for

example by reaction of a compound of formula (V) (as defined above) with an appropriate sulphur nucleophile in an analogous manner to the nucleophilic displacement reaction described with respect to process (C). If desired the above nucleophile may be used in the form of a metal thiolate salt.

When X in formula (V) is halogen, the reaction is preferably effected in the presence of an acid scavenging agent, for example a base such as triethylamine or calcium carbonate.

The reaction product may be separated from the reaction mixture, which may contain, for example, unchanged cephalosporin starting material and other substances, by a variety of processes including recrystallisation, ionophoresis, column chromatography and use of ion-exchangers (for example by chromatography on ion-exchange resins) or macroreticular resins.

A $\Delta^2$-cephalosporin ester derivative obtained in accordance with the process of the invention may be converted into the corresponding $\Delta^3$-derivative by, for example, treatment of the $\Delta^2$-ester with a base, such as pyridine or triethylamine.

Where a compound is obtained in which B is $>S \rightarrow O$ this may be converted into the corresponding sulphide by, for example, reduction of the corresponding acyloxysulphonium or alkoxysulphonium salt prepared _in situ_ by reaction with e.g. acetyl chloride in the case of an acetoxysulphonium salt, reduction being effected by, for example, sodium dithionite or by iodide ion as in a solution of potassium iodide in a water-miscible solvent e.g. acetic acid, acetone, tetrahydrofuran, dioxan, dimethylformamide or dimethylacetamide. The reaction may be effected at a temperature of from -20° to +50°C.

Metabolically labile ester derivatives of the compounds of formula (I) may be prepared by

reacting a compound of formula (I) or a salt or protected derivative thereof with the appropriate esterifying agent such as an acyloxyalkyl halide or alkoxycarbonyloxyalkyl halide (e.g. iodide) conveniently in an inert organic solvent such as dimethylformamide or acetone, followed, where necessary, by removal of any protecting groups.

Base salts of the compounds of formula (I) may be formed by reacting an acid of formula (I) with an appropriate base. Thus, for example, sodium or potassium salts may be prepared using the respective 2-ethylhexanoate or hydrogen carbonate salt. Acid addition salts may be prepared by reacting a compound of formula (I) or a metabolically labile ester derivative thereof with the appropriate acid.

Where a compound of formula (I) is obtained as a mixture of isomers, the syn isomer may be obtained by, for example, conventional methods such as crystallisation or chromatography.

For use as starting materials for the preparation of compounds of general formula (I) according to the invention, compounds of general formula (III) and the amide forming derivatives thereof such as acid halides and anhydrides corresponding thereto in their syn isomeric form or in the form of mixtures of the syn isomers and the corresponding anti isomers containing at least 90% of the syn isomer are preferably used.

Acids of formula (III) and their derivatives are themselves novel compounds and form a further feature of the invention. They may be prepared by etherification of a compound of formula

(VI)

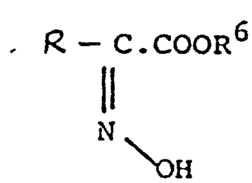

(wherein R is as hereinbefore defined and $R^6$ represents hydrogen or a carboxyl blocking group) or a salt thereof, by selective reaction with a compound of general formula

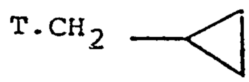

(VII)

(wherein T is a halogen atom, such as a chlorine, bromine or iodine atom; a sulphate group; or a sulphonate group, such as p-toluenesulphonate), followed by removal of any carboxyl blocking group $R^6$. Separation of isomers may be effected either before or after such etherification. The etherification reaction is conveniently carried out in the presence of a base, e.g. potassium carbonate or sodium hydride, and is preferably conducted in an organic solvent, for example dimethylsulphoxide, a cyclic ether such as tetrahydrofuran or dioxan, or an N,N-disubstituted amide such as dimethylformamide. Under these conditions the configuration of the oxyimino group is substantially unchanged by the etherification reaction. The reaction should be effected in the presence of a base if an acid addition salt of a compound of formula (VI) is used. The base should be used in sufficient quantity to neutralise rapidly the acid in question.

Acids of formula (III) may also be prepared by reaction of a compound of formula (VIII)

$R - CO.COOR^6$ (VIII)

(wherein R and $R^6$ are as hereinbefore defined) with a compound of formula (IX)

$H_2N.O.CH_2$ 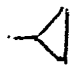 (IX)

followed by removal of any carboxyl blocking group $R^6$, and where necessary the separation of <u>syn</u> and <u>anti</u> isomers.

Compounds of formulae (VI) and (VIII) wherein R represents a group of formula

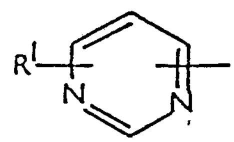

(wherein $R^1$ is as hereinbefore defined) may be prepared as described in UK Patent Application No. 2010840 A. Compounds of formulae (VI) and (VIII) wherein R represents a group of formula

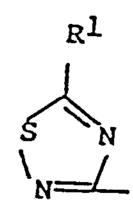

(wherein $R^1$ is as defined previously) may be prepared as described in European Patent Application No. 0013762.

Acids of formula (III) wherein R represents a group of formula

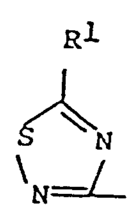

(wherein $R^1$ is as hereinbefore defined) may also be prepared by a method analogous to the process described in UK Patent Application No. 2094794 A. Thus, a halomethylcyclopropane can be reacted with a metal salt of a compound of formula.

$$(NC)_2C=NOH$$

and we have found the silver salt of this compound to be particularly convenient for this purpose. The resulting compound of formula

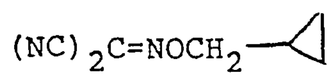

is then reacted with ammonia or a salt thereof to give a compound of formula

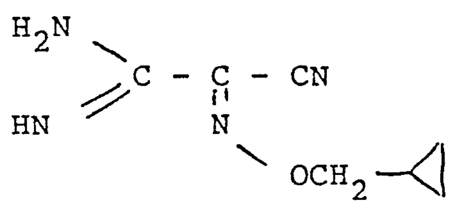

This compound is then halogenated and subsequently reacted with a thiocyanate salt, e.g. potassium thiocyanate, to give the compound

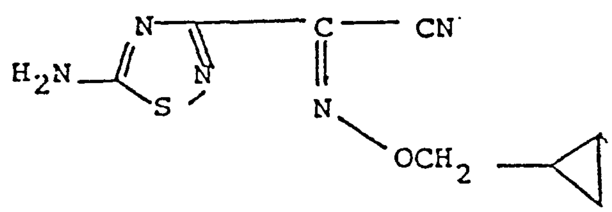

This compound may then be hydrolysed to give an acid of formula (III) in which R represents a 5-amino-1,2,4-thiadiazol-3-yl group and, if desired, the amino group may be protected.

Acids of general formula (III) as hereinbefore defined may be converted into the corresponding acid halides and anhydrides and acid addition salts by conventional methods, for example as described hereinabove.

Where X is a halogen atom in formula (V), ceph-3-em starting compounds may be prepared in conventional manner, e.g. by halogenation of a 7β-protected amino-3-methylceph-3-em-4-carboxylic acid ester 1β-oxide, removal of the 7β-protecting group, acylation of the resulting 7β-amino compound to form the desired 7β-acylamido group, e.g. in an analogous manner to process (A) above, followed by reduction of the 1β-oxide group later in the sequence. This is described in British Patent Specification No. 1326531. The corresponding ceph-2-em compounds may be prepared by the method of Dutch published Patent Application No. 6902013 by reaction of a 3-methylceph-2-em compound with N-bromosuccinimide to yield the corresponding 3-bromomethylceph-2-em-compound.

Compounds of formula (V) in which X represents an acyloxy group can be prepared by acylation of the corresponding 3-hydroxymethyl compounds in an analogous manner to process (B) above.

Compounds of formula (II) may also be prepared in conventional manner, e.g. by nucleophilic displacement of a corresponding 3-acyloxymethyl or 3-halomethyl compound with the appropriate nucleophile, e.g. as described in British Patent Specifications Nos. 1012943 and 1241657, and in British Patent Applications Nos. 2027691A, 2046261A and 2098216A.

Further references to the preparation of compounds of formula (II) include British Patents Specifications Nos. 1342241, 1361183, 1377762, 1435111, 1458293 and 1526978 and US Patent Nos. 3972774 and 4145538.

A further method for the preparation of the starting materials of formula (II) comprises deprotecting a corresponding protected 7β-amino compound, for example in conventional manner, e.g. using $PCl_5$.

It should be appreciated that in some of the above transformations it may be necessary to protect any sensitive groups in the molecule of the compound in question to avoid undesirable side reaction. For example, during any of the reaction sequences referred to above it may be necessary to protect the $NH_2$ group of the aminothiadiazolyl or aminopyrimidyl moiety, for example by tritylation, acylation (e.g. chloroacetylation, t-butoxycarbonylation or formylation), protonation or other conventional method, so that the protected group is stable to the reaction conditions accompanying one or more synthetic steps. The protecting group may thereafter be removed in any convenient way which does not cause breakdown of the desired compound, e.g. in the case of a trityl group by using an optionally halogenated carboxylic acid, e.g. acetic acid, formic acid, chloroacetic acid or trifluoroacetic acid or using a mineral acid, e.g. hydrochloric acid or mixtures of such acids, preferably in the presence of a protic solvent such as water, or, in the case of a chloroacetyl group, by treatment with thiourea.

Carboxyl blocking groups used in the preparation of compounds of formula (I) or in the preparation of necessary starting materials are desirably groups which may readily be split off at a suitable stage in the reaction sequence, conveniently at the last stage. It may, however, be convenient in some instances to employ non-toxic metabolically labile carboxyl blocking groups such as acyloxy-methyl or -ethyl groups (e.g. acetoxy-methyl or -ethyl or pivaloyloxymethyl) or alkoxycarbonyloxyalkyl groups (e.g. ethoxycarbonyloxyethyl) and retain these in the final product to give an appropriate ester derivative of formula (I).

Suitable carboxyl blocking groups are well known in the art, a list of representative blocked

carboxyl groups being included in British Patent No. 1399086. Preferred blocked carboxyl groups include aryl lower alkoxycarbonyl groups such as p-methoxybenzyloxycarbonyl, p-nitrobenzyloxycarbonyl and diphenylmethoxycarbonyl; lower alkoxycarbonyl groups such as t-butoxycarbonyl; and lower haloalkoxycarbonyl groups such as 2,2,2-trichloroethoxycarbonyl. The carboxyl blocking group may subsequently be removed by any of the appropriate methods disclosed in the literature; thus, for example, acid or base catalysed hydrolysis is applicable in many cases, as are enzymically-catalysed hydrolyses.

It will be appreciated that the use of amino protecting and carboxyl blocking groups is well known in the art and relevant examples of such use are given in e.g. Theodora W. Greene, "Protective Groups in Organic Synthesis" (Wiley-Interscience, New York, 1981), and J.F.W. McOmie, "Protective Groups in Organic Chemistry" (Plenum Press, London, 1973).

The antibiotic compounds of the invention may be formulated for administration in any convenient way, by analogy with other antibiotics and the invention therefore includes within its scope pharmaceutical compositions comprising an antibiotic compound in accordance with the invention adapted for use in human or veterinary medicine. Such compositions may be presented for use in conventional manner with the aid of any necessary pharmaceutical carriers or excipients.

The antibiotic compounds according to the invention may be formulated for injection and may be presented in unit dose form, in ampoules, or in multi-dose containers, if necessary with an added preservative. The compositions may also take such forms as suspensions, solutions, or emulsions in oily or aqueous vehicles, and may contain formulatory agents such as suspending, stabilising and/or dispersing

agents. Alternatively the active ingredient may be in powder form for reconstitution with a suitable vehicle, e.g. sterile, pyrogen-free water, before use.

If desired, such powder formulations may contain an appropriate non-toxic base in order to improve the water-solubility of the active ingredient and/or to ensure that when the powder is reconstituted with water, the pH of the resulting aqueous formulation is physiologically acceptable. Alternatively the base may be present in the water with which the powder is reconstituted. The base may be, for example, an inorganic base such as sodium carbonate, sodium bicarbonate or sodium acetate, or an organic base such as lysine or lysine acetate.

The antibiotic compounds may also for example be presented in a form suitable for absorption by the gastro-intestinal tract e.g. as tablets or capsules.

The antibiotic compounds may also, for example, be formulated as suppositories e.g. containing conventional suppository bases such as cocoa butter or other glycerides.

Compositions for veterinary medicine may, for example, be formulated as intramammary preparations in either long acting or quick-release bases.

The compositions may contain from 0.1% upwards, e.g. 0.1-99% of the active material, depending on the method of administration. When the compositions comprise dosage units, each unit will preferably contain 100-3000 mg e.g. 200-2000 mg of the active ingredient. The dosage as employed for adult human treatment will preferably range from 500 to 12000 mg e.g. 1000-10000 mg per day, depending on the route and frequency of administration. For example, in adult human treatment 500 to 4000 mg per day administered intravenously or intramuscularly will normally suffice. In treating <u>Pseudomonas</u> infections

higher daily doses may be required. It will be appreciated that in some circumstances, for example in the treatment of neonates, smaller dosage units and daily dosages may be desirable.

The antibiotic compounds according to the invention may be administered in combination with other therapeutic agents such as antibiotics, for example penicillins or other cephalosporins.

The following Examples illustrate the invention. All temperatures are in °C. DMSO is dimethylsulphoxide.

Preparation 1

2-(Cyclopropylmethyloxyimino)propanedinitrile

Bromomethylcyclopropane (14.851g) was added to a stirred solution of the silver salt of 2-(hydroxyimino)propanedinitrile (20.19g) in acetonitrile (450ml). A precipitate was formed immediately, and the suspension was stirred for 16 h at room temperature. The mixture was filtered, and the filtrate was evaporated to dryness under reduced pressure. The residue was triturated with diethyl ether, refiltered, and the filtrate re-evaporated. Distillation of the residue under reduced pressure gave the title compound as a colourless oil, b.p. 79-81°/2.0mm (9.517g).

Preparation 2

2-Cyano-2-(cyclopropylmethoxyimino)acetamidine

The product of Preparation 1 (9.0g) was added to a stirred cooled solution of ammonium chloride (6.455g) in 0.880 ammonia solution (50ml) and ethanol (50ml), maintained at -5°. The solution was stirred for 1.5h, maintaining the temperature at -5° to 0°, then diluted with water (100ml) and dichloromethane (100ml). The phases were separated, and the aqueous phase was extracted with more dichloromethane (2 x 50ml). The combined organic extracts were dried and evaporated to give the title compound (10.2g) as an unstable brown oil, which was used immediately in Preparation 3.

Preparation 3

(Z)2-(5-Amino-1,2,4-thiadiazol-3-yl)-2-cyclopropylmethoxyiminoacetonitrile

Bromine (3.0ml) was added dropwise to a stirred, cooled solution of the product of Preparation 2 (10.0g) and triethylamine (17ml) in methanol (120ml). The reaction temperature was maintained at -10° during the addition and for a further 5 min, when

a solution of potassium thiocyanate (5.684g) in methanol (60ml) was added dropwise, still keeping the reaction temperature at -10°. When addition was complete, the reaction mixture was stirred at 0° to 5° for 2h. The mixture was filtered, and the filtrate concentrated. Trituration of the residue gave the title compound as a solid (4.96g); $\nu_{max}$ (Nujol) 3445 and 3270 ($NH_2$), and 2240 $cm^{-1}$ (CN); τ (DMSO-$d_6$) values are 1.67 ($NH_2$, 5.69 ($CH_2$) and 9.2-9.72 ($C_3H_5$).

A further 2.27g of the title compound was obtained by extracting the aqueous filtrate with ethyl acetate, and chromatographing the extracts on silica gel, eluting with ethyl acetate: petrol (b.p. 40-60°) (1:1).

## Preparation 4

## (Z)-2-(5-Amino-1,2,4-thiadiazol-3-yl)-2-cyclopropylmethoxyimino-acetic acid

A stirred mixture of the product of Preparation 3 (4.757g) and sodium hydroxide (10g) in water (70ml) was heated at 55-60° for 7h. The resulting solution was cooled and filtered. The filtrate was acidified to pH 1.0 with concentrated hydrochloric acid, then extracted with ethyl acetate. The extracts were washed (water, brine), dried, and evaporated to give the title compound (4.789g) as a solid; $\lambda_{max}$ (ethanol) 236nm (ε 14000).

## Preparation 5

## Ethyl (Z)-2-Cyclopropylmethoxyimino-2-(4-formamido-pyrimid-2-yl) acetate.

A solution of ethyl 2-(4-formamido-pyrimid-2-yl)-2-hydroxyiminoacetate (preparation described in British Patent Application No. 2010840 A) (5.00g), cyclopropylmethyl bromide (3.2ml) and potassium carbonate (5g) in DMSO (50ml) was stirred under nitrogen for 1.5h. The resulting solution was

added dropwise to rapidly stirred water (400ml) and the product was extracted with ethyl acetate (5 x 200ml). The combined extracts were washed with water (5 x 200ml) and brine. Drying ($MgSO_4$) and removal of solvent gave a yellow solid, which was chromatographed on silica gel (150g), eluting with a mixture of dichloromethane and acetone (10:1 v/v) to give the title compound as an oily yellow solid (3.01g); $\nu_{max}$ ($CHBr_3$) 3385 (NH) and 1735 $cm^{-1}$ (ester).

Preparation 6

Methyl (Z)-2-cyclopropylmethoxyimino-2-(2-formamido-pyrimid-4-yl)acetate

The title compound was prepared according to the method of Preparation 5 starting from methyl (Z)-2-(2-formamidopyrimid-4-yl)-2-hydroxyiminoacetate. The title compound exhibited $\lambda_{max}$ (ethanol) 245.5nm ($\varepsilon$ 2900) and 203nm ($\varepsilon$ 5650).

Preparation 7

(Z)-2-(4-Aminopyrimid-2-yl)-2-cyclopropylmethoxyimino-acetic acid.

A solution of the product of Preparation 5 (2.49g) in ethanol (40ml) was treated with a solution of potassium hydroxide (1.43g) in water (3ml) and then stirred for 4h. The solution was acidified to pH2 with concentrated hydrochloric acid and filtered. The filtrate was evaporated to dryness and the residue was washed with ether, then dried in vacuo to give the title compound as a yellow solid (2.13g); $\lambda_{max}$ (ethanol) 261.5nm ($\varepsilon$ 15200).

Preparation 8

(Z)-2-(2-Aminopyrimid-4-yl)-2-cyclopropylmethoxyimino-acetic acid

The title compound was prepared according

to the method of Preparation 7 starting from the product of Preparation 6. The title compound exhibited $\lambda_{max}$ (ethanol) 242nm ($\varepsilon$ 15300) and 328.5nm ($\varepsilon$ 3450).

Example 1

(a) Diphenylmethyl (6R,7R)-7-[(Z)-2-(4-amino-pyrimid-2-yl)-2-cyclopropylmethoxyiminoacetamido]-3-carbamoyloxymethylceph-3-em-4-carboxylate.

A solution of phosphorus pentachloride (0.53g) in dichloromethane (10ml) was treated with the product of Preparation 7 at -20° under nitrogen. The resulting suspension was stirred for 20 minutes at -10°,after which, all the solid dissolved.

This solution was added in one portion to a solution of diphenylmethyl (6R,7R)-7-amino-3-carbamoyloxymethylceph-3-em-4-carboxylate (1.12g) and N,N-dimethylaniline (1.6ml) in dichloromethane (15ml) at -10°. The resulting suspension was stirred at -5° for 1h. Solvent was removed *in vacuo* and the residue added to ethyl acetate (100ml) and dilute hydrochloric acid (100ml). The resulting white solid was filtered, washed with water and ether, and dried *in vacuo*. Chromatography on silica gel (25g), eluting with a mixture of chloroform and acetone (1:1 v/v), gave the title compound as a white solid; $\nu_{max}$ (Nujol) 1775cm$^{-1}$ ($\beta$-lactam); $\tau$ (DMSO-d6) 0.57 (NH), 1.86 and 3.54 (pyrimidine), 2.4-2.75 (phenyl), 2.98 (amino-pyrimidine), 3.04 ($-C\underline{H}Ph_2$), 3.38 ($-CON\underline{H}_2$), 4.03 (7-H), 4.74 (6-H), 5.19-5.36, ($-C\underline{H}_2-OCONH_2$), 5.97 ($NOC\underline{H}_2$), 6.32-6.48 ($2-C\underline{H}_2$), 8.77 and 9.44-9.65 (cyclopropyl ring).

(b) (6R,7R)-7-[(Z)-2-(4-Aminopyrimid-2-yl)-2-cyclopropylmethoxyiminoacetamido]-3-carbamoyloxymethyl-ceph-3-em-4-carboxylic acid, mono-trifluoroacetic acid salt.

A solution of the product of Example 1(a) (0.49g) in anisole (2ml) was stirred with trifluoroacetic

acid (8ml) for 1h. The solution was then added dropwise to vigourously stirred water (200ml). The aqueous solution was washed with ether (4 x 75ml) then freeze-dried to give the title compound as a white powder (0.46g); $\lambda_{max}$ (ethanol) 244nm ($\varepsilon$ 24100); $\tau$(DMSO-d6) 0.44 (NH), 1.69 (-$NH_2$), 1.83 and 3.36 (pyrimidine) 3.42 (-CON$\underline{H}_2$), 4.14 (7-H), 4.80 (6-H), 5.08-5.34 (-C$\underline{H}_2$OCON$H_2$), 5.87 (NOC$\underline{H}_2$), 6.36 and 6.52 (2-C$\underline{H}_2$), 8.73 and 9.43-9.63 (cyclopropyl ring).

Example 2

(a) Diphenylmethyl (6R,7R)-7-[(Z)-2-(4-amino-pyrimid-2-yl)-2-cyclopropylmethoxyiminoacetamido]-3-methylceph-3-em-4-carboxylate

The title compound was prepared according to the method of Example 1(a) starting from diphenylmethyl (6R,7R)-7-amino-3-methylceph-3-em-4-carboxylate. The title compound exhibited $\lambda_{max}$ (ethanol) 245nm ($\varepsilon$ 24000); and $\tau$ (DMSO-d6) 0.59 (N$\underline{H}$), 1.86 and 3.55 (pyrimidine), 2.4-2.75 (phenyl), 2.98 (aminopyrimidine), 3.07 (C$\underline{H}$$Ph_2$), 4.10 (7-H), 4.79 (6-H), 5.97 (NOC$\underline{H}_2$), 6.35-6.57 (2-$CH_2$), 7.96 (methyl), 8.6-8.9 and 9.44-9.65 (cyclopropyl ring).

(b) (6R,7R)-7-[(Z)-2-(4-Aminopyrimid-2-yl)-2-cyclopropylmethoxyiminoacetamido]-3-methylceph-3-em-4-carboxylic acid, mono trifluoroacetic acid salt.

The title compound was prepared according to the method of Example 1(b) and exhibited $\lambda_{max}$ (ethanol), 244nm ($\varepsilon$ 25000); and $\tau$ (DMSO-d6), 0.49 (N$\underline{H}$), 1.60 (N$\underline{H}_2$), 1.82 and 3.38 (pyrimidine), 4.22 (7-H), 4.87 (6-H), 5.87 (NOC$\underline{H}_2$), 6.38 + 6.64 (2-C$\underline{H}_2$), 7.96 ($CH_3$), 8.74 and 9.45 -9.64 (cyclopropyl ring).

Example 3

a) Diphenylmethyl (6R,7R)-7-[(Z)-2-(2-aminopyrimid-

4-yl)-2-cyclopropylmethoxyiminoacetamido]-3-methylceph-3-em-4-carboxylate

The title compound was prepared according to the method of Example 2(a) starting from the product of Preparation 8. The title compound exhibited $\lambda_{max}$ (ethanol), 245nm ($\varepsilon$ 22500) and 332.5nm ($\varepsilon$ 4100); and $\nu_{max}$ ($CHBr_3$) 1780 ($\beta$-lactam), 1721 (ester) and 1683 $cm^{-1}$ (amide).

(b) (6R,7R)-7-[(Z)-2-(2-aminopyrimid-4-yl)-2-cyclopropylmethoxyiminoacetamido]-3-methylceph-3-em-4-carboxylic acid, mono trifluoroacetic acid salt

The title compound was prepared according to the method of Example 2(b), $\lambda_{max}$ (ethanol), 244.5nm ($\varepsilon$ 22600) and 330nm ($\varepsilon$ 3800); and $\nu_{max}$ (Nujol), 1761 ($\beta$-lactam), 1700 (acid) and 1663$cm^{-1}$ (amide).

Example 4

(a) Diphenylmethyl (6R,7R)-7-[(Z)-(2-aminopyrimid-4-yl)-2-cyclopropylmethoxyiminoacetamido]-3-carbamoyloxymethyl-ceph-3-em-4-carboxylate.

The title compound was prepared according to the method of Example 1(a) starting from the product of Preparation 8. The title compound exhibited $\lambda_{max}$ (ethanol), 245.5nm ($\varepsilon$ 22000) and 327.5 ($\varepsilon$ 3400); and $\nu_{max}$ ($CHBr_3$), 1785 ($\beta$-lactam), 1728 (ester + urethane) and 1685$cm^{-1}$ (amide).

(b) (6R,7R)-7-[(Z)-2-(2-aminopyrimid-4-yl)-2-cyclopropylmethoxyiminoacetamido]-3-carbamoyloxymethyl-ceph-3-em-4-carboxylic acid,mono trifluoroacetic acid salt.

The title compound was prepared according to the method of Example 1(b) and exhibited $\lambda_{max}$ (ethanol), 241nm ($\varepsilon$ 21000) and 324nm ($\varepsilon$ 3450); and $\nu_{max}$ (Nujol), 1780 ($\beta$-lactam), 1695 (acid + urethane) and 1665$cm^{-1}$ (amide).

Example 5

(6R,7R)-7-[(Z)-2-(4-aminopyrimid-2-yl)-2-cyclopropylmethoxyiminoacetamido]-3-pyridiniummethylceph-3-em-4-carboxylate

A solution of oxalyl chloride (0.235ml) in dichloromethane (3ml) was added dropwise to a solution of dimethylformamide (0.24ml) in dichloromethane (2ml) at -20° under nitrogen. The resulting white suspension was stirred in an ice-bath for 20 minutes then cooled to -20° and treated with the product of Preparation 7. The resulting yellow solution was stirred in an ice-bath for 10 minutes, then cooled to -20°, and added in one portion to a freshly prepared solution of (6R,7R)-7-amino-3-pyridiniummethylceph-3-em-4-carboxylate, dihydrochloride (1.02g) in water (3ml), triethylamine (1.5ml) and industrial methylated spirits (12ml). The resulting suspension was stirred for 1h and the solvents removed *in vacuo*. The residue was triturated with acetone and a yellow solid collected by filtration. This was chromatographed on silica gel (130g) eluting with mixtures of water and acetone. Fractions containing the product were combined and concentrated *in vacuo* to remove acetone. The aqueous solution was freeze-dried to yield the *title compound* as a yellow powder (0.795g), $\lambda_{max}$ (ethanol), 246.5nm ($\varepsilon$ 21200); $\tau$ (DMSO-d6), 0.52, 1.39 and 1.82 (pyridinium ring), 0.72 (NH), 1.92 and 3.59 (pyrimidine), 3.02 ($NH_2$), 4.26 (7-H), 4.32 and 4.81 ($CH_2$-$N^+$), 4.92 (6-H), 6.05 (NO$C\underline{H}_2$), 6.46 and 6.96 (2-$C\underline{H}_2$), 8.85 and 9.51 -9.72 (cyclopropyl ring).

Example 6

(6R,7R)-7-[(Z)-2-(2-aminopyrimid-4-yl)-2-cyclopropylmethoxyiminoacetamido]-3-pyridiniummethylceph-3-em-4-carboxylate.

The *title compound* was prepared according to the method of Example 5 starting from the product

of Preparation 8. The title compound exhibited $\lambda_{max}$ (ethanol), 237nm ($\varepsilon$ 21000) and 326nm ($\varepsilon$ 3600); and $\tau$ (DMSO-d6), 0.47, 1.37 and 1.79 (pyridinium), 0.52 (NH), 1.71 and 3.12 (pyrimidine), 3.36 ($NH_2$), 4.16 (7-H), 4.16 - 4.4 + 4.80($C\underline{H}_2$-N), 4.88 (6-H), 5.99 ($-OCH_2-$), 6.44 + 6.92 ($2-CH_2$), 8.84 and 9.47 - 9.70 (cyclopropyl ring).

Example 7

(a) Diphenylmethyl (6R,7R)-7-[(Z)-2-(5-amino-1,2,4,-thiadiazol-3-yl)-2-(cyclopropylmethoxyimino)acetamido]-3-carbamoyloxymethylceph-3-em-4-carboxylate.

To a stirred solution of phosphorus pentachloride (1.315g) in dichloromethane (20ml), cooled to -20°, was added the product of Preparation 4 (1.53g). The resulting solution was stirred for 15min at -10°, then added in one charge to a stirred, cooled (-10°) solution of diphenylmethyl (6R,7R)-7-amino-3-carbamoyloxymethylceph-3-em-4-carboxylate (2.775g) in dichloromethane (30ml) containing N,N-dimethylaniline (4.0ml). The solution was stirred at -10° to 0° for 2h. The solvent was removed *in vacuo*, and the residue was distributed between water and ethyl acetate. The organic phase was washed with 1N hydrochloric acid, water, saturated sodium bicarbonate solution, and saturated brine, dried, and evaporated to give the title compound as a yellow foam (3.033g); $\lambda_{max}$ (ethanol) 256.5nm ($\varepsilon$ 16300); and $\nu_{max}$ ($CHBr_3$), 1785 ($\beta$-lactam), 1730 ($CO_2-$), 1690$cm^{-1}$ (CONH).

(b) (6R,7R)-7-[(Z)-2-(5-Amino-1,2,4-thiadiazol-3-yl)-2-(cyclopropylmethoxyimino)acetamido]-3-carbamoyloxymethylceph-3-em-4-carboxylic acid

Water (2ml) was added to a solution of the product of Example 7(a) (1.0g) in formic acid (4ml), and the resulting solution was heated at 55° for 1h, then cooled, and evaporated to dryness. The residue was triturated with ether to give the title

compound as a pale-yellow foam (0.609g); $\lambda_{max}$ (pH6 buffer) 241nm ($\varepsilon$ 17900); $\nu_{max}$ (Nujol) 3420,3300 and 3200 (-NH + $-NH_2$), 1776 ($\beta$-lactam), 2600 and 1710 ($-CO_2H$), and 1670 and 1522 $cm^{-1}$ (-CONH-).

Example 8

(6R,7R)-7-[(Z)-2-(5-Amino-1,2,4-thiadiazol-3-yl)-2-(cyclopropylmethoxyimino)acetamido]-3-(1-pyridiniummethyl)ceph-3-em-4-carboxylate

Oxalyl chloride (0.24ml) was added to a stirred solution of N,N-dimethylformamide (0.24ml) in dichloromethane (7ml) maintained at -15°. The resulting suspension was stirred for 15 min at -15°, then the product of Preparation 4 (0.615g) was added to give an orange solution, which was stirred for 20 min at 0°, then cooled again to -20°. This cold solution was added in one charge to a solution of (6R,7R)-7-amino-3-(pyridiniummethyl)ceph-3-em-4-carboxylate dihydrochloride dihydrate (1.02g) in water (3ml) and ethanol (12ml) containing triethylamine (1.49ml). The mixture was stirred for one hour, then evaporated to dryness. The residue was triturated with water (50ml), filtered, and the filtrate freeze-dried. The solid residue was washed with dichloromethane, then chromatographed on silica (100g) eluting with acetone: water (9:1 to 7:3) to give the title compound as an off-white foam (0.315g); $\lambda_{max}$ (pH6 buffer), 247nm ($\varepsilon$ 22000); and $\nu_{max}$ (Nujol), 1775 ($\beta$-lactam), 1672 and 1528 (-CONH-) and 1610 $cm^{-1}$ ($CO_2^-$).

Example 9

(a) Diphenylmethyl (6R,7R)-7-[(Z)-2-(5-amino-1,2,4-thiadiazol-3-yl)-2-(cyclopropylmethoxyimino)acetamido]-3-(1-methyltetrazol-5-ylthiomethyl)ceph-3-em-4-carboxylate

The title compound was prepared according to the method of Example 7(a), and exhibited $\nu_{max}$

($CHBr_3$) 3490, 3395, 3315 and 3212 ($-NH_2$ + NH), 1787 (β-lactam), 1715 ($-CO_2$) and 1690 $cm^{-1}$ (CONH); τ ($CDCl_3$) values include 2.24 (NH), 3.89 (7-H), 4.91 (6-H), 5.78-6.00 ($CH_2$, cyclopropyl) and 9.4-9.8 ($C_3H_5$).

(b) (6R,7R)-7-[(Z)-2-(5-Amino-1,2,4-thiadiazol-3-yl)-2-(cyclopropylmethoxyimino)acetamido]-3-(1-methyltetrazol-5-yl-thiomethyl)ceph-3-em-4-carboxylic acid

A mixture of the product of Example 9(a) (0.632g), anisole (1.0ml), and trifluoroacetic acid (5ml) was stirred at ambient temperature for 10 min, then the resulting solution was added dropwise to rapidly stirred water (200ml). The precipitated solid was filtered off, washed with water and ether, and dried in vacuo to give the title compound (0.219g), $\lambda_{max}$ (pH6 buffer), 238nm (ε 21000); τ (DMSO-d6) values include 0.46 (-CONH), 4.17 (7-H), 4.85 (6-H), 5.61, 5.75 ($-CH_2S-$), 5.9-6.15 ($CH_2$, cyclopropyl) and 9.35-9.8 ($C_3H_5$).

CLAIMS

1. Cephalosporin compounds of general formula (I)

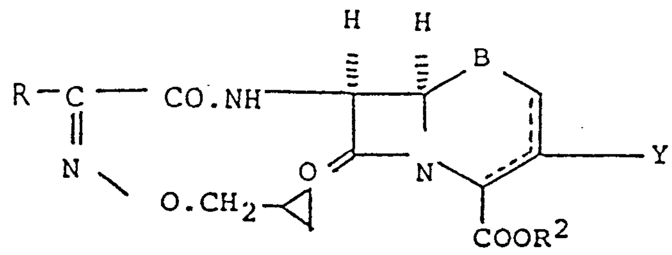

(I)

and salts thereof, wherein

R represents a group of formula

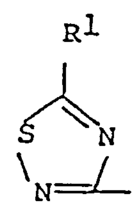

or

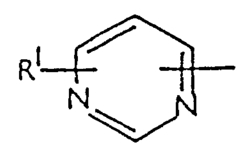

(wherein $R^1$ represents an amino or protected amino group);

$R^2$ represents a hydrogen atom or a carboxyl blocking group;

B is -S- or >S→O (α- or β-);

the dotted line bridging the 2-, 3- and 4-positions indicates that the compound is a ceph-2-em or ceph-3-em compound; and

Y represents a hydrogen or halogen atom; a $C_{1-4}$ alkoxy group; a methyl, ethyl or vinyl group; a $C_{1-4}$ alkylthio group; an acetoxymethyl group; a carbamoyloxymethyl group which may optionally be substituted on the nitrogen atom by a $C_{1-4}$ alkyl or $C_{1-4}$ haloalkyl group; an N-protected carbamoyloxymethyl group; a group of formula $-CH_2SZ$ (wherein Z is a carbon-linked 5- or 6-membered unsaturated heterocyclic ring containing at least one nitrogen atom, which ring may also contain one or more sulphur atoms and/or may be substituted by one or more

substituents independently selected from $C_{1-4}$ alkyl, oxo, hydroxy and carbamoylmethyl groups); a pyridiniummethyl group which may optionally be substituted by one or more substituents independently selected from halogen atoms and carbamoyl, hydroxymethyl and $C_{1-4}$ alkoxy groups; or a bicyclic pyridiniummethyl group of formula

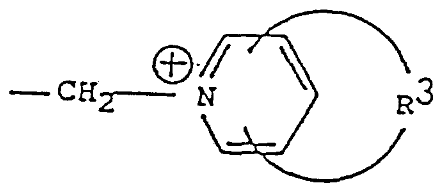

(wherein $R^3$ represents an optionally substituted $C_{2-5}$ hydrocarbon chain which is saturated or contains one or two double bonds and which may optionally be interrupted by a heteroatom),

with the proviso that, when R represents a 5-amino-1,2,4-thiadiazol-3-yl group, the group $-COOR^2$ is a carboxyl group, B is -S- and the dotted line represents $\Delta^3$ unsaturation, Y does not represent a 2,3-cyclopentenopyridiniummethyl group.

2. Cephalosporin antibiotics of general formulae

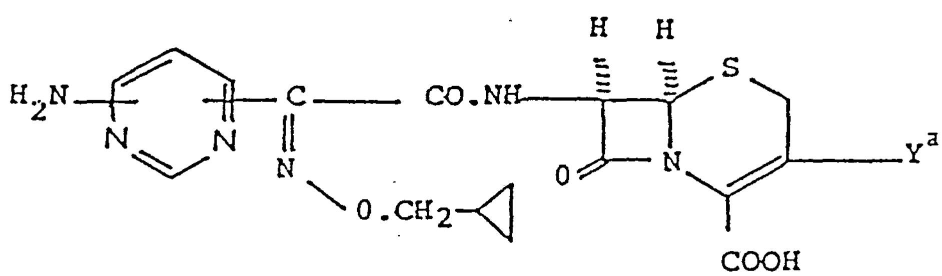

(Ia)

and

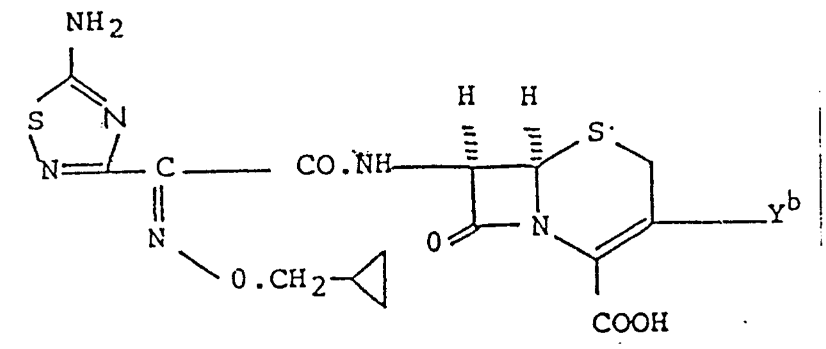

(Ib)

(wherein $Y^a$ respresents Y as defined in claim 1 other than an N-protected carbamoyloxymethyl group and $Y^b$ represents Y as defined in claim 1 other than a 2,3-cyclopentenopyridiniummethyl or an N-protected carbamoyloxymethyl group) and non-toxic salts and non-toxic metabolically labile esters thereof.

3. Compounds of general formula Ia as defined in claim 2 and non-toxic salts and non-toxic metabolically labile esters thereof, wherein the aminopyrimidyl group of the side chain at the 7β-position of the cephalosporic nuceleus is a 4-aminopyrimid-2-yl group.

4. Compounds as claimed in claim 2 or claim 3 wherein $Y^a$ or $Y^b$ represents a pyridiniummethyl or a carbamoyloxymethyl group.

5. A process for the preparation of compounds as claimed in claim 1, which comprises

(A) acylating a compound of the formula (II)

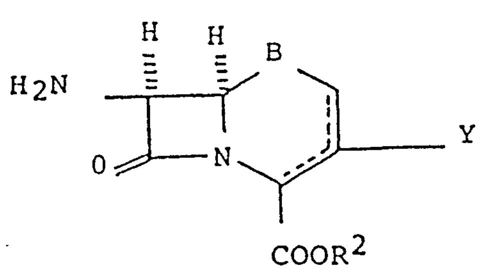

(II)

(wherein Y, B, the dotted line and $R^2$ are as defined in claim 1) or a salt or a 7-N-silyl derivative thereof, with an acid of formula (III)

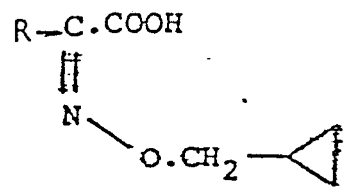

(III)

(wherein R is as defined in claim 1) or a salt thereof, or with an acylating agent corresponding thereto;

(B) for the preparation of compounds wherein Y represents an acetoxymethyl or optionally substituted carbamoyloxymethyl group, reacting a compound of formula (IV)

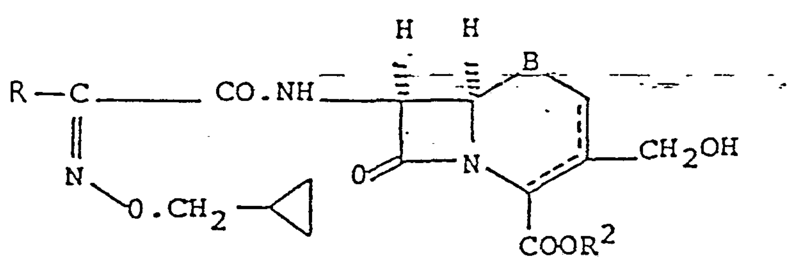

(IV)

(where R, $R^2$, B and the dotted line are as defined in claim 1) or a salt thereof, with an acylating agent serving to form the group $-CH_2Y'$ at the 3-position (wherein $Y'$ is an acetoxy group; a carbamoyloxy group optionally substituted on the nitrogen atom

by a $C_{1-4}$ alkyl or $C_{1-4}$ haloalkyl group; or an N-protected carbamoyloxy group);

(C) for the preparation of compounds wherein Y represents an optionally substituted pyridiniummethyl or bicyclic pyridiniummethyl group or a group of formula $-CH_2SZ$ (where Z is as defined in claim 1), reacting a compound of formula (V)

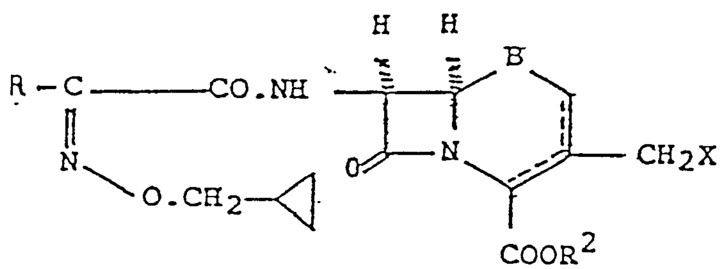

(V)

(wherein R, $R^2$, B and the dotted line are as defined in claim 1, and X represents a replaceable residue of a nucleophile) or a salt thereof, with a nucleophile serving to form the desired group Y at the 3-position; or

(D) for the preparation of compounds wherein Y represents a group of formula $-CH_2SZ$ where Z contains a $C_{1-4}$ alkyl-substituted or carbamoylmethyl-substituted nitrogen atom in the heterocyclic ring, reacting a compound of formula (I')

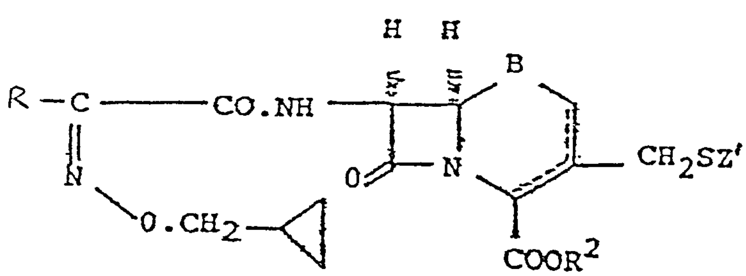

(I')

(wherein R, B and the dotted line are as defined in claim 1; $R^2$ represents a carboxyl blocking group; and Z' represents a carbon-linked 5- or 6-membered heterocyclic ring containing a nitrogen atom) with a $C_{1-4}$ alkylating agent or a carbamoylmethylating agent serving to introduce a $C_{1-4}$ alkyl group or a carbamoylmethyl group as a substituent on the nitrogen atom in the heterocyclic ring of Z'.

6. A process as claimed in claim 5 for the preparation of compounds according to claim 2, wherein, if necessary and/or desired in each instance, any of the following reactions, in any appropriate sequence, are carried out subsequent to reaction (A), (B), (C) or (D):

i) conversion of a $\Delta^2$-isomer into a $\Delta^3$-isomer,
ii) reduction of a compound wherein B is $>S\rightarrow O$ to form a compound wherein B is -S-,
iii) converion of a carboxyl group into a non-toxic metabolically labile ester function,
iv) formation of a non-toxic salt, and
v) removal of any carboxyl blocking and/or N-protecting groups.

7. A pharmaceutical composition comprising as active ingredient a compound according to claim 2 in association with one or more pharmaceutical carriers or excipients.

0150609

8. Compounds of general formulae (III), (IV) and (V) as defined in claim 5 and salts, esters and reactive derivatives thereof.